# EUROPEAN PATENT APPLICATION

(11) **EP 2 308 818 A1**
(43) Date of publication of application: **13.04.2011**
(21) Application number: 09171811.4
(22) Date of filing: 30.09.2009
(51) Int. Cl.: C07C 29/94

(54) **Process for the purification of crude alkaline glycerol**

(71) Applicant: Rhodia Poliamida E Especialidades Ltda, 05804-902 Jardim Sao Luiz - SP (ES)
(72) Inventor: Lourenco, Wagner, Celio, Ferraz, 130783-130 Campinas (SP) (BR); Macret, Richard, 01232-010 Soa Paulo (SP) (BR); Cielo, Jose, Eduardo, 13106-110 Sousas (SP) (BR)
(74) Representative: Boittiaux, Vincent

(57) **Abstract**

The present invention generally concerns a treatment process for crude alkaline glycerol obtained as a by-product of transesterification reaction of natural oils and fats with lower alcohols. The present invention particularly concerns the treatment of crude alkaline glycerol obtained as a by-product of the biodiesel production from vegetable oils and animal fats using organic alkyl carboxylic acids or their aqueous solution, comprising the steps of:
(a) acidification of the crude alkaline glycerol stock to pH in the range of about 4 to about 6, particularly 4 to 5, with organic alkyl carboxylic acids, in the presence of water in the range from about 5% to about 50%, particularly 15 to 30%, in weight with respect to the weight of the crude alkaline glycerol stock;
(b) separating the formed free fatty acids, for instance by flotation;
(c) removing the alcohol present in the acidified crude glycerol stock, for instance by distillation;
(d) separating the acidic glycerol.

## Description

The present invention generally concerns a treatment process for crude alkaline glycerol obtained as a by-product of transesterification reaction of natural oils and fats with lower alcohols. The present invention particularly concerns the treatment of crude alkaline glycerol obtained as a by-product of the biodiesel production from vegetable oils and animal fats using organic alkyl carboxylic acids or their aqueous solution.

### BACKGROUND OF THE INVENTION

There is a continuous and growing interest to produce chemicals from renewable sources which also include a great interest in the production of renewable fuels or bio fuels.

For instance, the Federal Government of Brazil launched in 2004 the National Program for the Production and use of Biodiesel, starting in January 2008 the addition of 2% of biodiesel to the diesel, known as B2, 3% (B3) in July/2008, 4% (B4) in July/2009 and being expected to increase the addition from 4% to 5% (B5) by 2010.

According to the National Petroleum Agency (ANP) of Brazil, the addition of 4% of biodiesel to the diesel represents an annual consumption of 2 billion liters for an installed capacity in January/2009 estimated in 3,7 billions of liters.

Presently 85% of the raw material for the production of biodiesel in the Brazilian biodiesel industry is soya bean oil, the remaining being from animal fat and other vegetable oils.

From a technological perspective, there are many references in the literature describing processes for the production of biodiesel. A basic procedure is described in the book from J. Van Gerpen and D. Clements, "Biodiesel Production Technology", August 2002-January 2004.

A known process for the production of biodiesel involves the transesterification of triglyceride-containing vegetable oils such as coconut, soy, castor, sunflower and peanut or animal fat, with a lower alkyl alcohols, in general methanol or ethanol, for instance in the presence of a homogeneous or heterogeneous alkaline catalyst.

In such a process, fatty acid alkyl esters are obtained from the triglycerides comprised in the natural oils and fats utilized as raw material, with the concomitant generation of crude glycerol as by-product. A general equation for that transesterification reaction would be:

triglyceride + 3 alkyl alcohol → glycerol + 3 fatty acid alkyl ester

Such triglycerides are transesterified with lower alkyl alcohols in the presence of an alkaline catalyst such as hydroxides or alkoxides, in a homogeneous or heterogeneous catalysis. Typically, an excess of alkyl alcohol is employed. After completion or the reaction, crude glycerol is separated from the biodiesel phase, for example by decantation. Usually 1000 kg of raw material results in 90% biodiesel and 10% crude alkaline glycerol.

The immiscible crude alkaline glycerol phase comprises a variable glycerol content as the major component, the excess alkyl alcohol, besides small amounts of fatty acid salts as soaps, some of the alkyl esters and the residual alkaline catalyst, which imparts an alkaline pH to the crude glycerol at this stage of the process.

Shown in table I below are typical samples of alkaline crude glycerol obtained from different production processes and starting raw materials such as soy oil and animal fat, with still large quantities remaining of the alcohol, in those cases methanol, alkaline catalyst used in the process and salts of free fatty acid in the form of soaps.

**Table I: Typical composition of alkaline crude glycerol from biodiesel processes.**

| ANALYSIS | Sample 1 | Sample 2 |
|---|---|---|
| pH at 25°C (10% w/w water solution) | 12,47 | 12,48 |
| Glycerol (% w/w) | 70,40 | 68,50 |
| Water (% w/w) | 0,40 | 0,42 |
| Methanol (% w/w) | 12,87 | 24,80 |
| Free Alkalinity (mg KOH/g) | 50,66 | 43,56 |
| Free Alkalinity calculated as sodium methoxide (% w/w) | 4,89 | 4,20 |
| Combined Alkalinity (mg KOH/g) | 2,86 | 2,76 |
| Combined Alkalinity calculated | | |
| as sodium linoleate (soaps) (% w/w) | 1,54 | 1,49 |
| Ash at 550°C (% w/w) | 5,30 | 4,40 |

This variation in the composition of crude alkaline glycerol obtained from biodiesel manufacturing processes, associated with the presence of impurities, especially residual alkyl alcohol and salts of free fatty acids (soaps) impose difficulties for the use of that glycerol as raw material for further industrial processes or its use as such.

A number of techniques for the treatment of alkaline crude glycerol have been described in the technical literature, typically comprising steps such as neutralization or acidification followed by conventional filtration and various other chemical treatments.

Typically in the process for biodiesel production via transesterification reaction an excess of the lower alkyl alcohol is used, making its recovery very important from an economics stand point.

The presence of soaps in the crude alkaline glycerol makes the recovery of the alcohol (for instance methanol) very difficult due the tendency for emulsification and foaming.

Despite of alcohol (ex: methanol) recovery, it is also desirable to remove or to recover the free fatty acids from the crude glycerol, produced during the transesterification reaction or already present in the starting material, and at this stage present as soaps, for instance by an acidification treatment.

To overcome this problem and also to recover the free fatty acids, a common step is the neutralization or sometimes acidification, either after the completion of the transesterification reaction or after the separation of the alkaline crude glycerol, often by using inorganic acids such as sulphuric, hydrochloric or phosphoric acid.

Depending on process conditions, the addition of acid makes possible the recovery of the alcohol by distillation without foaming, and conversion of soaps to free fatty acids that can be recovered and recycled or isolated for further transformations.

It is known that if either sulphuric or phosphoric acid are used to neutralize or acidify the alkaline crude glycerol, insoluble sulphate or phosphate salts are formed, requiring a laborious filtration step and sometimes a rinsing step with solvents for the removal of such salts. It is also known that if hydrochloric acid is used, soluble sodium chloride salt is formed, with corrosive effect on carbon steel and stainless steel therefore requiring special materials to further work with the crude glycerol that contains ionized chlorides.

The prior art discloses ways to deal with the acidification of crude alkaline glycerol.

The patent document US 2009/0137851 describes a process where the crude alkaline glycerol is treated, without prior neutralization, with a controlled percentage of water (about 1 %) and antifoaming agent to avoid excessive foaming during alcohol recovery by distillation. Afterwards, a very viscous alkaline glycerol (pH 12-13) is acidified with, for instance, 98% sulphuric acid and submitted to filtration or centrifugation.

In the patent document US 2009/0158640 the alkaline crude glycerol phase separated from the alkyl fatty acid esters can be acidulated to convert any soap to free fatty acids and be recovered. However, the treatment conditions such as the type of acid, range of pHs, presence of water, filtration, centrifugation etc... are not disclosed.

In the patent document US 2009/0178928, the alkaline crude glycerol is treated with hydrochloric acid, the free fatty acid separated by decantation, generating a glycerol solution containing 80-85% weight glycerol and 2,5% weight sodium chloride. The glycerol solution is diluted with water to 50% weight of glycerol and submitted to an electro dialysis procedure.

The patent document ES 2277727 describes new catalytic systems for the transesterification of triglycerides. In the example, after the alkyl fatty acid reaction is completed, the pH is adjusted to 7,0, while the reaction temperature is kept at 64 °C, using glacial acetic acid. After cooling on standing for three hours the two phases are separated. There is no mention to conditions for the recovery of alcohol or free fatty acids that might be present as soaps or any description of the crude glycerol quality obtained since the main objective of the patent is the use of a new catalytic system.

The patent document EP 1889899 describes a process for obtaining biodiesel through a combination of large number of unit operations aiming to the conversion of high free fatty acids feedstocks to glycerides and the subsequent conversion of glycerides to glycerin and fatty acid alkyl esters. In the neutralization of the transesterification catalyst in the reaction of triglycerides with alcohol, the use of an organic acid is mentioned, at a pH of 6.5-8.

### DESCRIPTION OF THE INVENTION

In view of that prior art, it is desirable to improve processes for the treatment of alkaline crude glycerol coming from industrial processes, particularly from the biodiesel production, by using organic acids and their aqueous solutions avoiding the presence of corrosive salts, such as sodium chloride, as well as additional steps, such as filtration and centrifugation needed for the elimination of insoluble salts such as sodium sulfate or phosphate providing an acidified crude glycerol having adequate quality for a range of further industrial transformations.

The present invention provides a process for the treatment of crude alkaline glycerol stock with the qualities above, as well as providing substantially no foaming caused by otherwise unreacted soaps contained in the glycerol.

The process of the invention, which is an improved process for the treatment of crude alkaline glycerol stock, comprises the steps of:
(a) acidification of the crude alkaline glycerol stock to pH in the range of about 4 to about 6, particularly 4 to 5, with organic alkyl carboxylic acids, in the presence of water in the range from about 5% to about 50%, particularly 15 to 30%, in weight with respect to the weight of the crude alkaline glycerol stock;
(b) separating the formed free fatty acids, for instance by flotation;
(c) removing the alcohol present in the acidified crude glycerol stock, for instance by distillation,
(d) separating the acidic glycerol.

The process of the invention is particularly advantageous for obtaining a chloride-free glycerol that is improved raw material for, for instance, the further production of glycerol esters mixtures (acetins), glycerol triacetate (triacetin), cetals and ketals by reaction with ketones or aldehydes, or purification by distillation or bidistillation, all without the need to use corrosion-resistant stainless steel.

By crude glycerol stock it is meant the glycerol that is a by-product of industrial processes, particularly alkaline crude glycerol recovered as a by-product from the biodiesel production from the transesterification of vegetable oils and/or animal fat with stoichiometric excess of lower alkyl alcohols, particularly methanol and ethanol.

The pH in step (a) is measured in a 10% weight/weight water solution at 25°C.

The process of this invention is suitable for the purification of alkaline crude glycerol stock with glycerol concentrations from 5 to 95% in weight, particularly in the typical range as obtained in the biodiesel production, such as from 40 to 90% glycerol, irrespective of the contents of alcohol (ex: methanol), alkaline catalyst, free fatty acids salts (soaps), the alkaline pH and other usual by-products from the biodiesel production process

By "aqueous organic alkyl carboxylic acids" it is meant that the acidification of alkaline crude glycerol stock in step (a) may be performed by the addition of substantially anhydrous alkyl carboxylic acids followed or preceded by addition of water, or by addition of aqueous solutions of such acids.

Without excluding any other, the organic alkyl carboxylic acids employed in the acidification of step (a) are particularly chosen from mono, di- and tri-acids. Adequate mono carboxylic acids are chosen from C1 to C6, more particularly C1 to C4 organic alkyl carboxylic acids such as formic, glacial acetic, propionic and butyric, and still more particularly, glacial acetic acid. Adequate di- and triacids are chosen from oxalic, maleic, succinic, glutaric, adipic and citric.

After the acidification step (a) to the pH range 4,0 to 5,0 and the presence of enough water concentration, the soaps are converted in free fatty acids and separate on the top of the glycerol solution in the reactor or decanter (flotation), according to the type of equipment used.

The use of organic alkyl carboxylic acids as acidifiers, for instance acetic acid, (to neutralize the alkaline catalyst used in the transesterification procedure that has glycerin as a by-product) avoids the formation of insoluble salts that require filtration, as well as the possible presence of corrosive soluble chloride salts. The specific pH range and the specific water content present in step (a) acidification of the invention promote the transformation of soaps in fatty acids that can be separated from the glycerol - either a different pH range or a different water content range let the soluble soaps be carried on in the resulting glycerol.

In fact, experiments run by the applicant showed that the mere addition of acetic acid in crude alkaline glycerol stock, if pH is not between 4 and 6 and a certain amount of water is not present, the fatty acids do not separate at the top of the glycerol layer when a reactor, a tank or a decanter is used for the acidification step.

It was also verified by the applicant that when pH of the crude glycerol stock is between 4 and 6, but the water content is too low, the liberated fatty acids remain practically soluble and cannot be recovered at the top of glycerol layer when a reactor, a tank or a decanter is used for the acidification step.

With the prescribed pH and water ranges, the lower alkyl alcohols (for instance methanol) can be recovered by distillation without the formation of foam.

Despite of the temperature of acidification step (a) not being a limiting factor for the invention, it can be carried out at the usual temperatures employed in the process for the separation of crude alkaline glycerol from the alkyl fatty acid esters, such as 100 to 60 °C or at lower temperatures such as from 60 to 20 C.

The process of the invention may be performed in batch operations as well as a continuous process.

The water content of the final crude glycerol solution is variable and controlled by the step (c) of methanol recovery by distillation. Typical compositions of the glycerol solution obtained by the process of this invention, after water addition, adjustment of pH to 4,0 with glacial acetic acid, recovery of free fatty acid on top of the glycerol layer when a reactor, a tank or a decanter is used for the acidification step and recovery of methanol by distillation are shown on Table II.

**Table II: Composition of crude glycerol after treatment with glacial acetic acid**

| ANALYSIS | Sample 1 | Sample 2 | Sample 3 |
|---|---|---|---|
| pH at 25°C (10% w/w water solution) | 6,15 | 5,20 | 5,30 |
| Glycerol (% w/w) | 87,8 | 80,1 | 79.5 |
| Water (% w/w) | 1,4 | 9,1 | 8,5 |
| Methanol (% w/w) | * | * | * |
| Free Alkalinity (mg KOH/g) | * | * | * |
| Combined Alkalinity (meq/g) | 1,010 | 0,924 | 0,946 |
| Ash at 550°C (% w/w) | 5,70 | 5,11 | 5,10 |

| | | | |
|---|---|---|---|
| (*) = below the detection limit | | | |

### EXAMPLES

The following examples are given only to show adequate embodiments of the invention, and do not in any way limit the invention beyond the content of the claims presented further on.

### Example 1

In order to verify if filtration could be avoided in the acidification step of alkaline crude glycerol, the crude glycerol described on Table I (sample 1) was treated with mixtures of 98% sulfuric acid and glacial acetic acid.

In all experiments an insoluble solid was obtained and filtration was necessary. Despite of the filtration operation, the salt formed was partially removed by filtration as a solid and part remained soluble in the glycerol solution as analyzed by the ash content. It was also observed that there was a great retention of glycerol in the filtrated solid which needs to be removed by washing (Tables III and IV).

**Table III: Mixtures of 98% sulfuric and acetic acids - Acidification of alkaline crude glycerol**

| Test | 98% Sulfuric Acid (%) | Glacial Acetic Acid (%) | Alkaline Crude Glycerol (g) | Water Added (g) | Acidic Mixture Added (g) | Final Weight (g) | Final pH |
|---|---|---|---|---|---|---|---|
| 1 | 100 | 0 | 500 | 93 | 28,3 | 621,3 | 2 |
| 2 | 90 | 10 | 500 | 93 | 26,7 | 619,7 | 4 |
| 3 | 80 | 20 | 500 | 93 | 24,9 | 617,9 | 4 |
| 4 | 50 | 50 | 500 | 93 | 33,8 | 626,8 | 4 |
| 5 | 20 | 80 | 500 | 93 | 42 | 635,0 | 4 |

**Table IV: Analytical results for the acidification of alkaline crude glycerol after filtration**

| | Liquid + Solid | Liquid after Filtration | | Solid after filtration | |
|---|---|---|---|---|---|
| Test | Total Weight (g) | Weight (g) | Ash (% w/w) | Weight (g) | Ash (% w/w) |
| 1 | 621,3 | 513,4 | 2,28 | 65,6 | 29,04 |
| 2 | 619,7 | 509,6 | 1,87 | 64,2 | 34,06 |
| 3 | 617,9 | 508,3 | 1,88 | 73,6 | 27,72 |
| 4 | 626,8 | 515,2 | 2,11 | 72 | 25,79 |
| 5 | 635,0 | 546,5 | 3,14 | 43,5 | 21,02 |

Comments: As can be seen, the invention does not use inorganic acidifying acids, and advantageously, therefore avoiding further operations to obtain an acidified crude glycerol, in a more efficient process.

### EXAMPLE 2

A comparative result concerning the characteristics of crude glycerol solution obtained when 33% hydrochloric acid and glacial acetic acid were used in the crude alkaline glycerol treatment shown on Table V.

The alkaline crude glycerol of sample 2 of table I (1000 g) was heated to 78 to 82 °C and the methanol partially distilled at atmospheric pressure (186,8 g). Water was added (161 g) followed by the acidification with 33% hydrochloric acid (82,2g) until pH of the solution equals to about 2,0. The solution was allowed to stand for 45 minutes during which time a solid formed on the top of the solution (31,7 g). The liquid phase was separated by decantation, a 50% sodium hydroxide solution was added until pH of the solution in the range of 6,0 to 7,0. The solution was heated to 80 to 85 °C at atmospheric pressure for the distillation of methanol containing some water (71,1 g). The crude glycerol solution obtained (736,2 g) was a brownish clear liquid with 90,5% weight of glycerol and showed the composition described on Table V, sample 2.

**Table V: Composition of crude glycerol after treatment with glacial acetic acid and 33% hydrochloric acid**

| ANALYSIS | Sample 1 Acetic Acid | Sample 2 33% Hydrochloric Acid |
|---|---|---|
| pH at 25°C (10% w/w water solution) | 6,15 | 3,59 |
| Glycerol (% w/w) | 87,80 | 90,50 |
| Water (% w/w) | 1,40 | 1,20 |
| Methanol (% w/w) | * | * |
| Free Alkalinity (mg KOH/g) | * | * |
| Combined Alkalinity (meq/g) | 1,01 | * |
| Chloride (% w/w) | * | 3,97 |
| Sodium Chloride (% w/w) | * | 6,54 |
| Ash at 550°C (% w/w) | 5,70 | 4,40 |

| | | |
|---|---|---|
| (*) = below the detection limit | | |

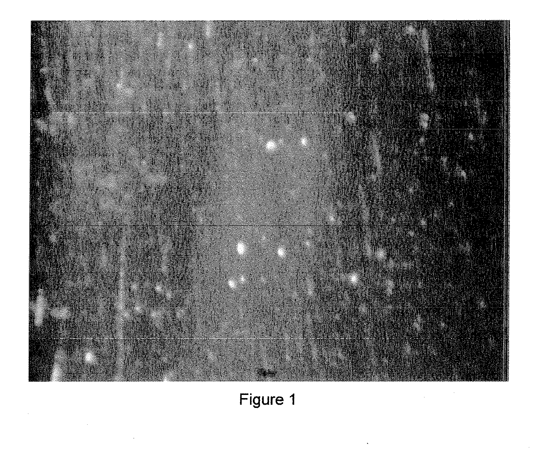

The glycerol solution obtained by acidification with 33% hydrochloric acid (sample 2 - Table V) was submitted to immersion tests with austenitic stainless steel during 30 days and it was observed the pitting nucleation as shown in figure 1 - a stereoscopy photography of an austenitic stainless steel surface showing bright spots of pitting nucleation.

COMMENTS - It can be seen that the invention avoids the problems of using hydrochloric acid to neutralize the alkaline catalyst present in the crude glycerol stock.

### EXAMPLES 3 to 6

In those examples, it can be seen that the crude acidified glycerol obtained by the use of glacial acetic acid presents a similar composition as the acidification by hydrochloric acid but with a higher final pH and complete absence of chloride as shown on Table V.

### EXAMPLE 3

The alkaline crude glycerol of sample 2 of table I (500 g) was heated to 78 to 85 °C and the methanol partially distilled at atmospheric pressure (123 g). Water was added (84 g) followed by the acidification with glacial acetic acid (25,3g) until the pH of the solution in the range of 4,0 to 5,0. The solution was allowed to stand for 60 minutes during which time a solid formed on the top of the solution (22,5 g). The liquid phase was separated by decantation and heated to 90 to 95 °C at atmospheric pressure for the distillation of methanol containing some water (20,4 g). The crude glycerol solution obtained (444,5 g) was a brownish clear liquid with 87,8 % weight of glycerol and showed the composition described on Table II, sample 1.

### Example 4

Glacial acetic acid was added to the alkaline crude glycerol of sample 1 of table I (700 g) until the pH of the solution is about 7,0, without addition of water. The solution was allowed to stand for 1 hour and no solid on top of the glycerol layer was observed. The addition of glacial acetic acid was continued stopping in pH = 6,0, 5,0 and 4,0, with intervals of 1 hour between additions and no solid on top of the glycerol layer was observed. To this solution with pH 4,0 it was added water (140 g) and after 60 minutes standing, a solid was formed on top of the glycerol layer.

### Example 5

Glacial acetic acid was added to the alkaline crude glycerol of sample 1 of table I (500 g) until the pH of the solution is about 4,0. Water was added (40g) and the solution let to stand for 90 minutes. There was no formation of solid on top of the glycerol layer. Additional water was added (40 g) and turbidity was noted, therefore additional water was added (40 g) and after 30 minutes standing a solid starts to form on top of the reactor. The liquid phase was separated by decantation and heated to 90 to 95 °C at atmospheric pressure for the distillation of methanol containing some water. The crude glycerol solution obtained was slightly colored clear liquid with 80,1% weight of glycerol and showed the composition described on Table II, sample 2.

### Example 6

The alkaline crude glycerol of sample 2 of table I (15 Kg) was heated to 79 to 82 oC and the methanol partially distilled at atmospheric pressure. When the temperature of the reaction mass reaches about 90 °C some foaming was observed and the distillation stopped collecting (2,87 Kg) of methanol. Water was added (2,4 Kg) followed by the acidification with glacial acetic acid (0,877 Kg) until the pH of the solution in the range of 4,0 to 5,0. Stirring of the solution was kept at 60 °C for 1 hour and allowed to stand for 30 minutes during which time a solid formed on the top of the solution (0,42 Kg). The liquid phase was separated by decantation and heated to 90 to 95 °C at atmospheric pressure for the distillation of methanol containing some water (1,74 Kg). The crude glycerol solution obtained (11,64 Kg) showed 79,5 % weight of glycerol with the composition described on Table II, sample 3.

It is well understood that a person skilled in the art, with the help of the teachings brought herein, is able to perform embodiments of the invention not expressly described in this text, with substantially the same function to reach substantially the same results, but such procedure is equivalent to the invention, therefore being encompassed by the claims presented further on.

## Claims

1. Process for the treatment of crude alkaline glycerol stock, **characterized by** the fact that it comprises the steps of:
(a) acidification of the crude alkaline glycerol stock to pH from about 4 to about 6, with organic alkyl carboxylic acids, in the presence of water in the range from about 5% to about 50%, in weight with respect to the weight of the glycerol stock;
(b) separating the formed free fatty acids;
(c) removing the alcohol present in the crude acidified glycerol stock,
(d) separating the acidic glycerol.

2. Process, according to claim 1, **characterized by** the fact that said crude alkaline glycerol stock is a by-product of transesterification reaction of natural oils and fats with lower alkyl alcohols, particularly crude alkaline glycerol obtained as a by-product of the biodiesel production from vegetable oils and animal fats using an stoichiometric excess of lower alkyl alcohols.

3. Process, according to any of the preceding claims, **characterized by** the fact that said acidification is to pH from 4 to 5.

4. Process, according to any of the preceding claims, **characterized by** the fact that said water range is from 15 to 30%.

5. Process, according to any of the preceding claims, **characterized by** the fact that the separation of free fatty acids in step (b) is by flotation.

6. Process, according to any of the preceding claims, **characterized by** the fact that the removal of alcohol in step (c) is distillation.

7. Process, according to any of the preceding claims, **characterized by** the fact that said crude alkaline glycerol stock comprises glycerol concentrations from 5 to 95% in weight, particularly from 40 to 90%.

8. Process, according to any of the preceding claims, **characterized by** the fact that the acidification of alkaline crude glycerol stock in step (a) is chosen from the addition of substantially anhydrous alkyl carboxylic acids followed or preceded by addition of water, or by addition of aqueous solutions of such acids.

9. Process, according to any of the preceding claims, **characterized by** the fact that the organic alkyl carboxylic acids employed in the acidification of step (a) are chosen from mono, di- and tri-acids.

10. Process, according to any of the preceding claims, **characterized by** the fact that the organic alkyl carboxylic acids employed in the acidification of step (a) are monoacids chosen from C1 to C6, more particularly C1 to C4 organic alkyl carboxylic acids.

11. Process, according to claim 10, **characterized by** the fact that said monoacids are chosen from formic, acetic, propionic and butyric.

12. Process, according to claim 10 **characterized by** the fact that one said monoacid is glacial acetic acid.

13. Process, according to claim 9, **characterized by** the fact that the organic alkyl carboxylic acids employed in the acidification of step (a) are chosen oxalic, maleic, succinic, glutaric, adipic and citric.

14. Process, according to any of the preceding claims, **characterized by** the fact step (a) is performed at temperatures from 100 to 20°C.
